# EUROPEAN PATENT APPLICATION

(11) **EP 4 778 550 A1**
(43) Date of publication of application: **22.07.2026**
(21) Application number: 24864797.6
(22) Date of filing: 11.10.2024
(51) Int. Cl.: A61M 5/178, A61M 5/31, A61M 5/315

(54) **DOSING DEVICE CAPABLE OF SETTING AND DIALING BACK DOSE**

(30) Priority: 11.09.2023 CN 202311166088
(71) Applicant: Shanghai Bravo Technology Co., Ltd, Shanghai 201605 (CN)
(72) Inventor: WU, Ding, Shanghai 201605 (CN); GUO, Rong, Shanghai 201605 (CN)
(74) Representative: karo IP
(86) International application number: PCT/CN2024/124110
(87) International publication number: WO 2025/056082

(57) **Abstract**

A drug delivery device capable of setting and recalling a dose is provided, which includes a shell. The following components are mounted inside the shell: a push rod, which is configured to move to deliver a drug according to a set dose during injection; a rotor, which is configured to be rotated during injection to drive the push rod to axially move forwards; a rotating shaft, which is configured to drive the rotor to rotate during injection, and can cooperate with the push rod in the axial movement process to control final dose setting; a differential nut, which is configured to implement differential adjustment and drive the rotating shaft to axially move; the rotatable tube for dose setting, which can drive the bushing to rotate during injection; and the bushing, which is configured to drive the rotating shaft to rotate during injection.

## Description

### TECHNICAL FIELD

The present disclosure belongs to the technical field of drug delivery devices, and specifically to, a drug delivery device capable of setting and recalling a dose.

### BACKGROUND

With the development of insulin pen technology, current requirements for an insulin pen include: (1) The insulin pen can inject insulin with a small dose for multiple times. Generally, insulin can be injected at a maximum single dose (60 IU) for no less than five times. (2) It is convenient to adjust a single dose within a range of 0 to 60 IU, and adjustment accuracy is high (up to 1 IU). (3) During adjustment of a dose, a user can see scales, or since the insulin pen can make sounds, it is convenient for patients with poor vision or even blind patients to inject insulin on their own. (4) During injection, the operation is simple. The insulin pen can also make a sound in the injection process to indicate that the injection is in progressing.

Existing CN103170038A discloses an insulin pen which includes a pen cap, a needle, an injection pipe, an injection cannula, a locating fixture block, a screw, a nut pipe, a fixing pipe, a rotating pipe, and a button. A piston is arranged in the injection pipe, and insulin is put into the injection pipe in advance. Partial threads on two sides of the screw are respectively cut off in an axial direction, so that two sections which are parallel to each other are formed. A pressing head is arranged at a front end of the screw. The screw is coupled with an inner thread of the nut pipe. The insulin pen further includes a rotating handle and a rotary spring. The rotating handle is arranged at an outer end, namely, a side opposite to the pen cap, of the insulin pen. An outer end of the rotating pipe is arranged inside the rotating handle and is fixed on the rotating handle. Another end of the rotating pipe is clamped with the nut pipe. The locating fixture block sleeves the rotating pipe and is clamped with the rotating pipe. The nut pipe is arranged between the pressing head and the locating fixture block. The rotary spring is sleeved on the rotating pipe. One end of the rotary spring is fixed on an inner side of the rotating handle, and another end of the rotary spring is fixed on the locating fixture block. By repeated adjustment on the locating fixture block, the rotary spring, and a clutch spring, the insulin pen can be used for multiple times, and can precisely control metered insulin injection. Although the insulin pen can precisely control a dose, its adjustment and control structure is relatively complex, cannot recall a dose, and cannot prevent sub-dose injection.

### SUMMARY

For the problems in the background section, the present disclosure aims to provide a drug delivery device capable of setting and recalling a dose, which has a simple and proper structure, can recall a dose, and can prevent sub-dose injection.

Technical solutions employed in the present disclosure are as follows:
A drug delivery device capable of setting and recalling a dose includes a cartridge holder. A removable pen cap is arranged at a front end of the cartridge holder. A cartridge is mounted inside the cartridge holder. A rear end of the cartridge holder is connected with a shell. The following components are mounted inside the shell:
a push rod, which is configured to move to deliver a drug according to a set dose during injection;
a rotor, which is in threaded connection with the push rod, is configured to be rotated during injection to drive the push rod to axially move forwards, and can cooperate with the shell for injection sound production;
a rotating shaft, a front end of which is connected into a rear end of the rotor, where the rotating shaft is configured to drive the rotor to rotate during injection, can axially move relative to the rotor, and can cooperate with the push rod in the axial movement process to control final dose setting;
a differential nut, which is mounted on an outer side of a rear end of the rotating shaft and is configured to: implement differential adjustment and drive the rotating shaft to axially move;
a differential screw, which is fixed inside the shell and is arranged between the differential nut and a rotatable tube for dose setting, where the differential screw is in threaded connection with both the differential nut and the rotatable tube for dose setting for differential adjustment, and is rotatably connected to the rotor to axially limit the rotor;
the rotatable tube for dose setting, which is configured to drive a bushing to axially move during dose setting and cooperates with the bushing for dose setting sound production, where the rotatable tube for dose setting can be meshed with the bushing during injection and drive the bushing to rotate, and is configured to drive the differential nut to synchronously rotate; and
the bushing, which is connected to an inner side of the rear end of the rotating shaft and is configured to drive the rotating shaft to rotate during injection; and
an injection button capable of applying a force to drive the rotatable tube for dose setting to counterclockwise rotate and drive the bushing and the rotatable tube for dose setting to synchronously rotate after the bushing and the rotatable tube for dose setting are meshed is mounted inside a rear end of the rotatable tube for dose setting.

Further, a unidirectional ratchet that protrudes is arranged on an outer surface of the rotor; rabbets are annularly and uniformly distributed inside the shell; and during rotation, the unidirectional ratchet cooperates with the rabbets for injection sound production.

Further, a meshing tooth is arranged in an axial direction of each of the rotatable tube for dose setting and the bushing; and when the rotatable tube for dose setting is rotated, the meshing tooth on the rotatable tube for dose setting and the meshing tooth on the bushing are in sliding fit for dose setting sound production.

Further, the rotor and the rotating shaft are in axial slot connection; the rotating shaft and the bushing are in axial slot connection; and the differential nut and the rotatable tube for dose setting are in axial slot connection.

Further, the rotating shaft and the differential nut are in circumferential ring connection; and the rotor and the differential screw are in circumferential ring connection.

Further, a flange is arranged at a rear end of the push rod; a step is arranged inside a front end of the rotating shaft; and the flange is in contact with the step for limitation to control final dose setting.

Further, the push rod is a screw.

Further, a push plate for cooperating with a piston of the cartridge is mounted at a front end of the push rod.

Further, the push rod and the push plate are of an integrated structure, and a through slot that radially penetrates through the front end is provided in the front end of the push rod.

Further, a spring for resetting is arranged between the injection button and the bushing.

Compared with the prior art, the present disclosure has outstanding advantages including:
1. The rotating shaft can cooperate with the push rod in the axial movement process to control the final dose setting and prevent sub-dose injection.
2. Dose setting and recalling processes and an injection process are implemented by cooperation of the rotatable tube for dose setting, the differential screw, the differential nut, the rotating shaft, the bushing, and the rotor, and the structure is simple and proper.
3. The rotor cooperates with the shell to implement injection sound production to remind a user of an injection dose.
4. The rotatable tube for dose setting cooperates with the bushing to produce a sound to remind a user of dose setting.

### BRIEF DESCRIPTION OF THE DRAWINGS

FIG. 1 is a schematic structural diagram of an appearance of the present disclosure;
FIG. 2 is a schematic diagram of an exploded structure according to the present disclosure;
FIG. 3 is a schematic diagram of a cross-sectional structure of the present disclosure;
FIG. 4 is a schematic structural diagram of dose setting according to the present disclosure;
FIG. 5 is a schematic structural diagram of an injection process according to the present disclosure;
FIG. 6 is a schematic structural diagram of controlling of final dose setting according to the present disclosure;
FIG. 7 is a schematic structural diagram of the present disclosure after use.
FIG. 8 is a schematic structural diagram of a rotor and a shell cooperating with each other to produce a sound according to the present disclosure;
FIG. 9 is a schematic diagram of axial slot connection between a differential nut and a rotatable tube for dose setting according to the present disclosure; and
FIG. 10 is a second schematic structural diagram of the present disclosure.

In the drawings: 1: pen cap; 2: cartridge holder; 3: cartridge; 4: rotor; 41: unidirectional ratchet; 42: ring slot; 5: push plate; 6: shell; 61: rabbet; 7: differential screw; 71: ring rib; 8: push rod; 81: flange; 82: through slot; 9: rotating shaft; 91: step; 10: differential nut; 101: rib; 11: rotatable tube for dose setting; 111: elongated slot; 12: bushing; 13: injection button; 14: spring; and 15: meshing tooth.

### DETAILED DESCRIPTION OF THE EMBODIMENTS

The present disclosure will be further described below with reference to specific embodiments, but is not limited to these specific implementations. A person skilled in the art may understand that the present disclosure encompasses all alternatives, improvements, and equivalences that are possibly included within the scope of the claims.

In the descriptions of the present disclosure, it should be understood that orientations or positional relationships indicated by the technical terms "center", "longitudinal", "transverse", "length", "width", "thickness", "upper", "lower", "front", "rear", "left", "right", "vertical", "horizontal", "top", "bottom", "inner", "clockwise", "anticlockwise", and the like are orientations or positional relationships as shown in the drawings, and are only for the purpose of facilitating and simplifying the descriptions of the present disclosure instead of indicating or implying that devices or elements indicated must have particular orientations, and be constructed and operated in the particular orientations, so that these terms are not construed as limiting the present disclosure. In addition, the terms "first" and "second" are used for descriptive purposes only and are not to be construed as indicating or implying relative importance or to implicitly indicate the number of technical features indicated. From this, features defined as "first" and second" may explicitly or implicitly include one or more features. In the description of the present disclosure, "plurality" means two or more, unless otherwise expressly and specifically defined.

In the present disclosure, unless otherwise expressly specified and limited, the terms "mount", "link", "connect", "fix", and the like should be understood in a broad sense, such as, a fixed connection, a detachable connection, an integrated connection, a mechanical connection, an electrical connection, a direct connection, an indirect connection through an intermediate medium, and an internal communication of two elements. A person of ordinary skill in the art may understand the specific meanings of the foregoing terms in the present application according to specific situations.

In the present disclosure, unless otherwise explicitly stipulated and restricted, that a first feature is "on" or "under" a second feature may include that the first and second features are in direct contact, or may include that the first and second features are in indirect contact through another feature. In addition, that the first feature is "on", "above", or "over" the second feature includes that the first feature is directly or diagonally above the second feature, or merely indicates that a level of the first feature is greater than that of the second feature. That the first feature is "below", "beneath", and "under" of the second feature indicates that the first feature is directly or diagonally below the second feature, or merely indicates that a level of the first feature is less than that of the second feature.

### Embodiment I

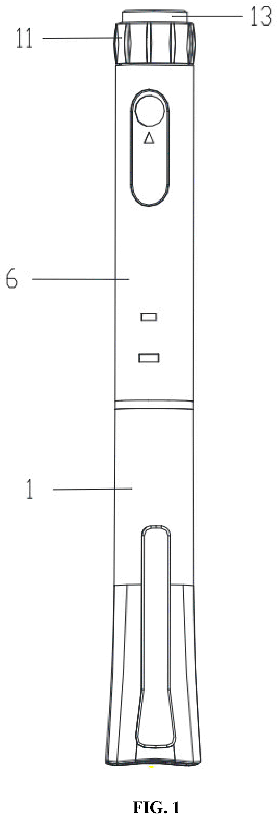

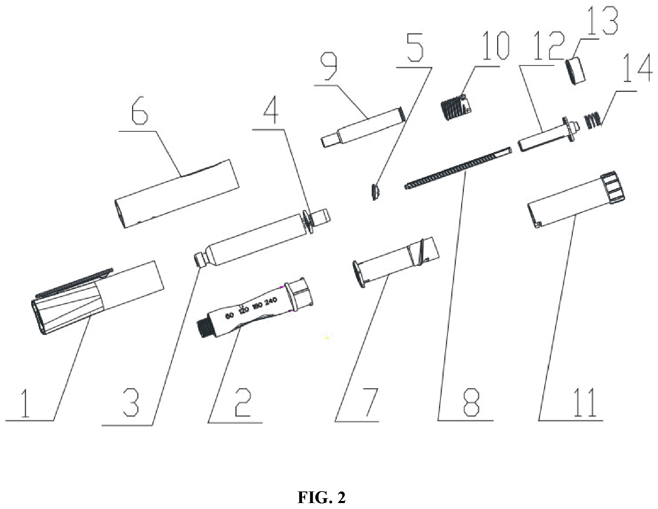

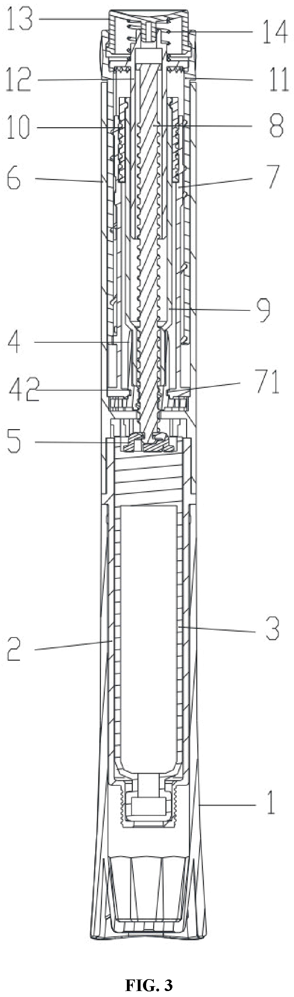

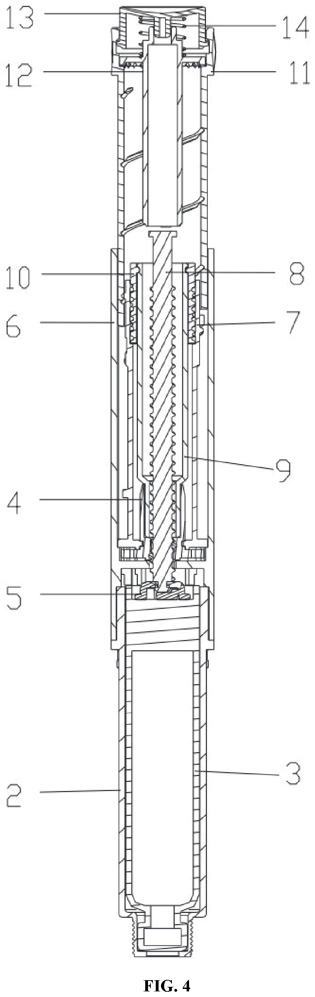

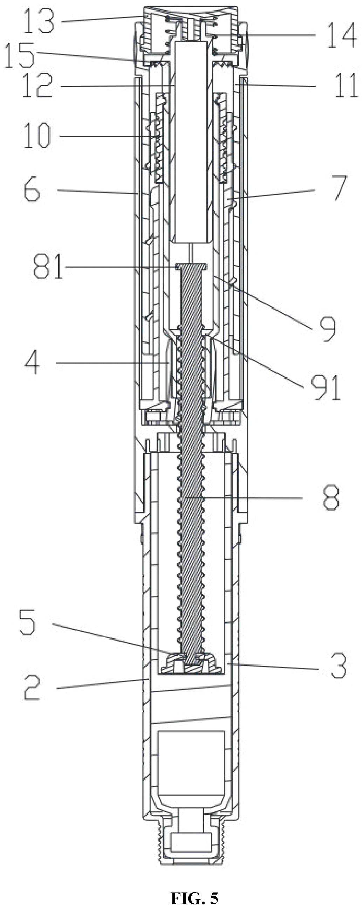

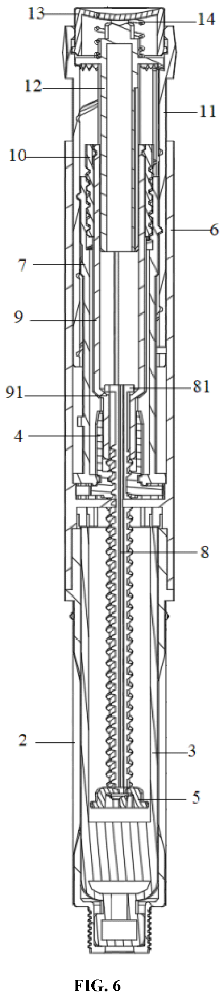

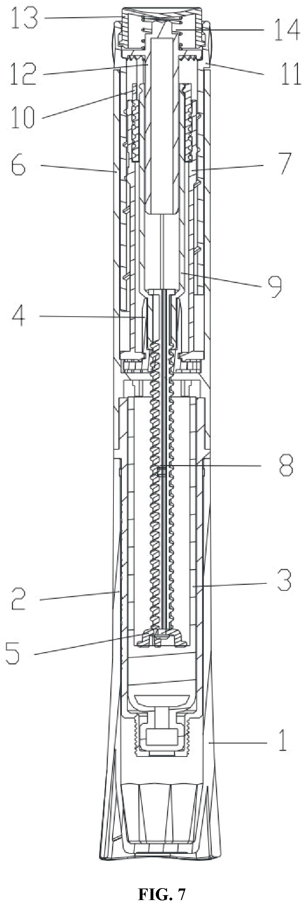

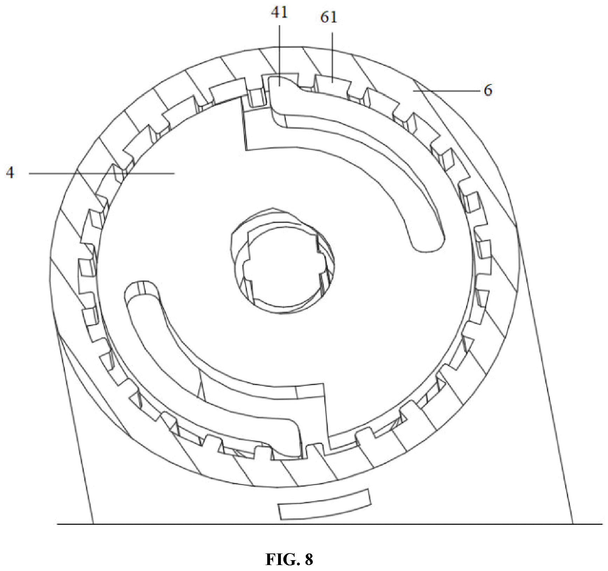

Referring to FIG. 1 to FIG. 9, this embodiment provides a drug delivery device capable of setting and recalling a dose, including a cartridge holder 2. A removable pen cap 1 is arranged at a front end of the cartridge holder 2. A cartridge 3 is mounted inside the cartridge holder 2. A rear end of the cartridge holder 2 is connected with a shell 6. The following components are mounted inside the shell 6:
a push rod 8, which is a screw structure and configured to move to deliver a drug according to a set dose during injection, where a push plate 5 for cooperating with a piston of the cartridge 3 is mounted at a front end of the push rod 8;
a rotor 4, which is in threaded connection with the push rod 8, is configured to be rotated during injection to drive the push rod 8 to axially move forwards, and can cooperate with the shell 6 for injection sound production;
a rotating shaft 9, a front end of which is connected into a rear end of the rotor 4, where the rotating shaft is configured to drive the rotor 4 to rotate during injection, can axially move relative to the rotor 4, and can cooperate with the push rod 8 in the axial movement process to control final dose setting;
a differential nut 10, which is mounted on an outer side of a rear end of the rotating shaft 9 and is configured to: implement differential adjustment and drive the rotating shaft 9 to axially move;
a differential screw 7, which is fixed inside the shell 6 and is arranged between the differential nut 10 and a rotatable tube 11 for dose setting, where the differential screw 7 is in threaded connection with both the differential nut 10 and the rotatable tube 11 for dose setting for differential adjustment, and is rotatably connected to the rotor 4 to axially limit the rotor 4, so that the rotor can radially rotate only instead of axially moving, and thread pitches of internal and external threads of the differential screw 7 are different, so that differential adjustment is implemented;
the rotatable tube 11 for dose setting, which is configured to drive a bushing 12 to axially move during dose setting and cooperates with the bushing 12 for dose setting sound production, where the rotatable tube 11 for dose setting can be meshed with the bushing 12 during injection and drive the bushing 12 to rotate, and is configured to drive the differential nut 10 to synchronously rotate; and
the bushing 12, which is connected to an inner side of the rear end of the rotating shaft 9 and is configured to drive the rotating shaft 9 to rotate during injection.

An injection button 13 capable of applying a force to drive the rotatable tube 11 for dose setting to counterclockwise rotate and drive the bushing 12 and the rotatable tube 11 for dose setting to synchronously rotate after the bushing 12 and the rotatable tube 11 for dose setting are meshed is mounted inside a rear end of the rotatable tube 11 for dose setting. A spring 14 for resetting is arranged between the injection button 13 and the bushing 12.

In this embodiment, a unidirectional ratchet 41 that protrudes is arranged on an outer surface of the rotor 4. Rabbets 61 are annularly and uniformly distributed inside the shell 6. During rotation, the unidirectional ratchet 41 cooperates with the rabbets 61 for injection sound production. The unidirectional ratchet 41 restrains the rotor 4 to counterclockwise rotate only. It can be set that a prompt sound can be produced for every unit of rotation, to remind a user of an injection dose. Specifically, 24 rabbets 61 are provided inside the shell 6. The unidirectional ratchet 41 is symmetrically arranged on the outer surface of the rotor 4. A sound is produced when the rotor 4 is rotated 15 degrees.

In this embodiment, a meshing tooth 15 is arranged in an axial direction of each of the rotatable tube 11 for dose setting and the bushing 12. When the rotatable tube 11 for dose setting is rotated, the meshing tooth 15 on the rotatable tube 11 for dose setting and the meshing tooth 15 on the bushing 12 are in sliding fit for dose setting sound production. The meshing tooth 15 on the rotatable tube 11 for dose setting and the meshing tooth 15 on the bushing 12 are axially abutted. When the rotatable tube 11 for dose setting is rotated, vibrations and sounds are produced because of direct collision between tooth surfaces.

In this embodiment, the rotor 4 and the rotating shaft 9 are in axial slot connection; the rotating shaft 9 and the bushing 12 are in axial slot connection; and the differential nut 10 and the rotatable tube 11 for dose setting are in axial slot connection, thereby implementing synchronous rotation. The axial slot connection between the differential nut 10 and the rotatable tube 11 for dose setting is used as an example. A protruding rib 101 or rib base is axially arranged on an outer surface of the differential nut 10, and an elongated slot 111 is axially provided in an inner wall of the rotatable tube 11 for dose setting. The rib 101 and the elongated slot 111 cooperate for axial slot connection.

The rotating shaft 9 and the differential nut 10 are in circumferential ring connection; and the rotor 4 and the differential screw 7 are in circumferential ring connection. The rotor 4 and the differential screw 7 are used as an example. A ring slot 42 is provided in an outer surface of the rotor 4, and a protruding ring rib 71 or rib base is arranged on an inner surface of the differential screw 7. The ring slot 42 and the ring rib 71 cooperate for circumferential ring connection. The circumferential ring connection between the rotor 4 and the differential screw 7 is to limit axial movement of the rotor 4. The rotor 4 can be rotated relative to the differential screw 7. The circumferential ring connection between the rotating shaft 9 and the differential nut 10 is to drive the rotating shaft 9 to axially move through the differential nut 10. The rotating shaft 9 and the differential nut 10 can be rotated relatively.

In this embodiment, a flange 81 is arranged at a rear end of the push rod 8. A step 91 is arranged inside a front end of the rotating shaft 9. The flange 81 is in contact with the step 91 for limitation to control final dose setting.

In the present disclosure, during dose setting, the rotatable tube 11 for dose setting is clockwise rotated. Due to the axial slot connection between the rotatable tube 11 for dose setting and the differential nut 10, the differential nut 10 can synchronously clockwise rotate along with the rotatable tube 11 for dose setting.

The differential nut 10 is in threaded connection with the fixed differential screw 7, so that the differential nut 10 can clockwise rotate along a threaded track of the differential screw 7 in the above rotation process and axially move towards a near end. The differential nut 10 and the rotating shaft 9 are in circumferential ring connection, so that the rotating shaft 9 can be driven to axially move towards the near end. The rotor 4 and the rotating shaft 9 are in axial slot connection, and the rotating shaft 9 and the bushing 12 are in axial slot connection. The rotor 4 is always limited in the axial direction and is configured to unidirectionally counterclockwise rotate only. Therefore, during dose setting, the rotor 4 does not rotate and restrains the rotating shaft 9 from rotating. The rotating shaft 9 then restrains the bushing 12 from rotating. Because of the abutment relationship between a near end of the rotatable tube 11 for dose setting and the bushing 12, the bushing 12 can only follow the rotatable tube 11 for dose setting to axially move towards the near end. During this period, at an abutment position between the rotatable tube 11 for dose setting and the bushing 12, vibrations and sounds are produced as one tooth surface scratches another tooth surface, to implement dose setting sound production.

In the present disclosure, for recalling, a recalling process and the dose setting process are inverse processes. The rotatable tube 11 for dose setting is counterclockwise rotated, and the differential nut 10 can follow the rotatable tube 11 for dose setting to synchronously counterclockwise rotate and can counterclockwise rotate along the threaded track of the differential screw 7 and axially move towards a far end, thereby implementing recalling. In the recalling process, the rotating shaft 9 follows the differential nut 10 to axially move towards the far end. The bushing 12 follows the rotatable tube 11 for dose setting to axially move towards the far end under the limitation by the injection button 13 and the spring 14. During this period, at the abutment position between the rotatable tube 11 for dose setting and the bushing 12, vibrations and sounds are produced as one tooth surface scratches another tooth surface, to implement dose recalling sound production.

In the present disclosure, for injection, the injection button 13 is pushed, and a force is applied to the rotatable tube 11 for dose setting. Meanwhile, the rotatable tube 11 for dose setting and the bushing 12 are in tooth surface meshing. The rotatable tube 11 for dose setting is counterclockwise rotated towards the far end, and the differential nut 10 that is in axial slot connection with the rotatable tube 11 for dose setting synchronously counterclockwise rotates towards the far end. The bushing 12 also synchronously counterclockwise rotates. The bushing 12 drives the rotating shaft 9 to counterclockwise rotate, and the rotating shaft 9 drives the rotor 4 to counterclockwise rotate. The rotor 4 drives the push rod 8 to linearly move towards the far end, thereby implementing injection. The rotor 4 can produce a prompt sound for every unit of rotation. Because the differential nut 10 and the rotating shaft 9 are in circumferential ring connection, the differential nut 10 can drive the rotating shaft 9 to move towards the far end in the injection process.

In the present disclosure, during the controlling of the final dose setting, the rotatable tube 11 for dose setting is clockwise rotated. The differential nut 10 that synchronously clockwise rotates drives the rotating shaft 9 to axially move towards the near end. When the step 91 arranged inside the rotating shaft 9 is abutted against the flange 81 of the push rod 8, the rotating shaft 9 cannot continue to move towards the near end. Due to the circumferential ring connection between the rotating shaft 9 and the differential nut 10, if the rotating shaft 9 cannot move towards the near end, the differential nut 10 cannot move towards the near end either. Due to the axial slot connection between the differential nut 10 and the rotatable tube 11 for dose setting, the rotatable tube 11 for dose setting cannot clockwise rotate either. In this case, if a predetermined dose cannot be reached by continuously rotating the rotatable tube 11 for dose setting in current dose setting, the current dose setting fails, and an insulin pen user is clearly prompted. Thus, a final dose is controlled, and occurrence of sub-dose injection is prevented.

In the present disclosure, the rotating shaft 9 can cooperate with the push rod 8 in the axial movement process to control the final dose setting and prevent sub-dose injection. The dose setting and recalling processes and the injection process are implemented by cooperation of the rotatable tube 11 for dose setting, the differential screw 7, the differential nut 10, the rotating shaft 9, the bushing 12, and the rotor 4, and the structure is simple and proper. The rotor 4 cooperates with the shell 6 to implement injection sound production to remind a user of an injection dose. The rotatable tube 11 for dose setting cooperates with the bushing 12 to produce a sound to remind a user of dose setting.

### Embodiment II

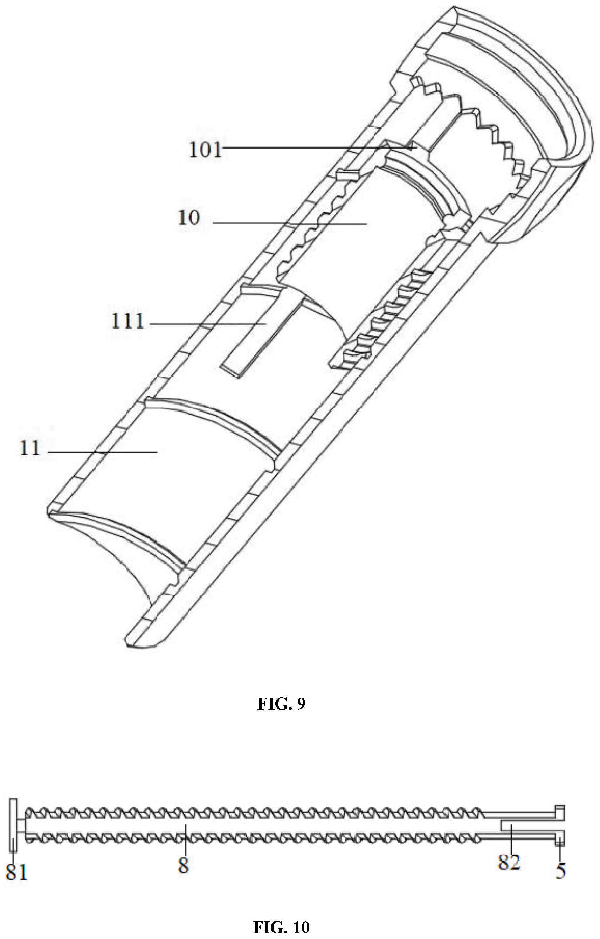

Referring to FIG. 10, a difference between this embodiment and Embodiment I is that the push rod 8 and the push plate 5 are of an integrated structure, and a through slot 82 that radially penetrates through the front end is provided in the front end of the push rod 8. It is convenient for deformation and assembling during assembling. Meanwhile, the number of components is reduced, and an assembling process is simplified.

For other structures and functions, refer to the description in Embodiment I.

## Claims

1. A drug delivery device capable of setting and recalling a dose, comprising a cartridge holder (2), wherein a removable pen cap (1) is arranged at a front end of the cartridge holder (2); a cartridge is mounted inside the cartridge holder (2); a rear end of the cartridge holder (2) is connected with a shell (6); the following components are mounted inside the shell (6):
a push rod (8), which is configured to move to deliver a drug according to a set dose during injection;
a rotor (4), which is in threaded connection with the push rod (8), is configured to be rotated during injection to drive the push rod (8) to axially move forwards, and is able to cooperate with the shell (6) for injection sound production;
a rotating shaft (9), a front end of which is connected into a rear end of the rotor (4), wherein the rotating shaft is configured to drive the rotor (4) to rotate during injection, is able to axially move relative to the rotor (4), and is able to cooperate with the push rod (8) in the axial movement process to control final dose setting;
a differential nut (10), which is mounted on an outer side of a rear end of the rotating shaft (9) and is configured for differential adjustment and to drive the rotating shaft (9) to axially move;
a differential screw (7), which is fixed inside the shell (6) and is arranged between the differential nut (10) and a rotatable tube (11) for dose setting, wherein the differential screw (7) is in threaded connection with both the differential nut (10) and the rotatable tube (11) for dose setting for differential adjustment, and is rotatably connected to the rotor (4) to axially limit the rotor (4);
the rotatable tube (11) for dose setting, which is configured to drive a bushing (12) to axially move during dose setting and cooperates with the bushing (12) for dose setting sound production, wherein the rotatable tube (11) for dose setting is able to be meshed with the bushing (12) during injection and drive the bushing (12) to rotate, and is configured to drive the differential nut (10) to synchronously rotate; and
the bushing (12), which is connected to an inner side of the rear end of the rotating shaft (9) and is configured to drive the rotating shaft (9) to rotate during injection; and
an injection button (13) capable of applying a force to drive the rotatable tube (11) for dose setting to counterclockwise rotate and drive the bushing (12) and the rotatable tube (11) for dose setting to synchronously rotate after the bushing (12) and the rotatable tube (11) for dose setting are meshed is mounted inside a rear end of the rotatable tube (11) for dose setting.

2. The drug delivery device capable of setting and recalling the dose according to claim 1, wherein a unidirectional ratchet (41) that protrudes is arranged on an outer surface of the rotor (4); rabbets (61) are annularly and uniformly distributed inside the shell (6); and during rotation, the unidirectional ratchet (41) cooperates with the rabbets (61) for injection sound production.

3. The drug delivery device capable of setting and recalling the dose according to claim 1, wherein a meshing tooth (15) is arranged in an axial direction of each of the rotatable tube (11) for dose setting and the bushing (12); and when the rotatable tube (11) for dose setting is rotated, the meshing tooth (15) on the rotatable tube (11) for dose setting and the meshing tooth (15) on the bushing (12) are in sliding fit for dose setting sound production.

4. The drug delivery device capable of setting and recalling the dose according to claim 1, wherein the rotor (4) and the rotating shaft (9) are in axial slot connection; the rotating shaft (9) and the bushing (12) are in axial slot connection; and the differential nut (10) and the rotatable tube (11) for dose setting are in axial slot connection.

5. The drug delivery device capable of setting and recalling the dose according to claim 1, wherein the rotating shaft (9) and the differential nut (10) are in circumferential ring connection; and the rotor (4) and the differential screw (7) are in circumferential ring connection.

6. The drug delivery device capable of setting and recalling the dose according to claim 1, wherein a flange (81) is arranged at a rear end of the push rod (8); a step (91) is arranged inside a front end of the rotating shaft (9); and the flange (81) is in contact with the step (91) for limitation to control final dose setting.

7. The drug delivery device capable of setting and recalling the dose according to any one of claims 1 to 6, wherein the push rod (8) is a screw.

8. The drug delivery device capable of setting and recalling the dose according to claim 7, wherein a push plate (5) for cooperating with a piston of the cartridge (3) is mounted at a front end of the push rod (8).

9. The drug delivery device capable of setting and recalling the dose according to claim 8, wherein the push rod (8) and the push plate (5) are of an integrated structure, and a through slot (82) that radially penetrates through the front end is provided in the front end of the push rod (8).

10. The drug delivery device capable of setting and recalling the dose according to claim 1, wherein a spring (14) for resetting is arranged between the injection button (13) and the bushing (12).
